# EUROPÄISCHE PATENTSCHRIFT

(11) **EP 2 513 285 B1**
(45) Veröffentlichungstag und Bekanntmachung des Hinweises auf die Patenterteilung: **29.10.2014**
(21) Anmeldenummer: 10801381.4
(22) Anmeldetag: 16.12.2010
(51) Int. Cl.: C12M 1/107, B01F 7/00, B01F 7/02, B01F 7/06

(54) **BEHÄLTER EINER BIOGASANLAGE SOWIE VERFAHREN ZUR ENTNAHME EINER KOMPONENTE AUS DEM BEHÄLTER**
CONTAINER OF A BIOGAS SYSTEM AND METHOD FOR REMOVING A COMPONENT FROM THE CONTAINER
RÉCIPIENT D'UNE INSTALLATION DE BIOGAZ ET PROCÉDÉ POUR PRÉLEVER UN COMPOSANT DU RÉCIPIENT

(30) Priorität: 16.12.2009 DE 102009060115
(43) Veröffentlichungstag der Anmeldung: 24.10.2012
(73) Patentinhaber: MT-Energie GmbH, 27404 Zeven (DE)
(72) Erfinder: MARTENS, Christoph, 27404 Ostereistedt (OT Rockstedt) (DE)
(74) Vertreter: Hauck Patent- und Rechtsanwälte
(86) Internationale Anmeldenummer: PCT/EP2010/007665
(87) Internationale Veröffentlichungsnummer: WO 2011/082787

(56) Entgegenhaltungen:
- EP-A1- 1 992 405
- DE-A1- 3 408 454
- DE-A1-102007 038 692
- DE-U1-202009 010 167

## Beschreibung

Die Erfindung betrifft einen Behälter einer Biogasanlage, umfassend eine aus dem Behälter entnehmbare Komponente, beispielsweise ein Rührwerk zum Mischen von in dem Behälter befindlichem Substrat, eine Entnahmeöffnung, sowie eine Entnahmeeinrichtung, mit der die Komponente beispielsweise für Wartungszwecke zumindest teilweise durch die Entnahmeöffnung aus dem Behälter entnommen werden kann. Außerdem betrifft die Erfindung ein Verfahren zur zumindest teilweisen Entnahme einer Komponente, beispielsweise eines Rührwerks zum Mischen von in einem Behälter einer Biogasanlage befindlichem Substrat, aus dem Behälter durch eine Entnahmeöffnung des Behälters beispielsweise für Wartungszwecke.

Fermentations- und Lagerbehälter von Biogasanlagen sind mit entnehmbaren Komponenten, wie Rührwerken, ausgestattet. Je nach Ausgestaltung können in einem Behälter ein oder mehrere Rührwerke vorgesehen sein. Bekannte Rührwerke können sich in ihrer Art erheblich unterscheiden. Eine grobe Unterscheidung kann zwischen Rührwerken mit in dem Behälter liegendem (abgetauchtem) Antriebsaggregat (Tauchmotorrührwerken) einerseits und Rührwerken mit außen liegendem Antriebsaggregat andererseits getroffen werden. Weitere Merkmale der Rührwerke sind z.B. Propellergröße und Drehzahl eines zum Rühren eingesetzten Propellers. Sogenannte "Paddelgiganten" sind meist extern angetriebene, stark untersetzte Paddelrührwerke, die durch langsame Paddelbewegungen in dem Behälter für einen kontinuierlichen Mischvorgang sorgen. Klassische Tauchmotorrührwerke verfügen dagegen über einen verhältnismäßig kleinen Propellerdurchmesser zwischen beispielsweise 0,6 m und 1,2 m und werden zum Mischen der Substrate in der Regel im Intervallbetrieb und mit hohen Drehzahlen betrieben. Dazwischen existieren noch sogenannte Großflügelrührwerke, die mit einem Propellerdurchmesser zwischen z.B. 1,6 m bis 3 m in einem mittleren Drehzahlbereich betrieben werden. Hierbei kann der Rührvorgang kontinuierlich oder im Intervallbetrieb erfolgen. Grundsätzlich erfolgt der Antrieb wahlweise über ein externes oder in dem Behälter angeordnetes Antriebsaggregat.

Bekannt sind außerdem Domrührwerke, die an der Decke des Behälters befestigt werden. In der Regel wird ein Großflügelrührwerk oder ein Paddelrührwerk von oben durch eine Behälterdecke in den Behälter eingeführt und zumindest von einem außerhalb des Behälters sitzenden Antriebsaggregat angetrieben. Beschrieben sind solche Rührwerke beispielsweise in DE 20 2004 004 101 U1 und DE 20 2006 020 195 Ul. Auch bekannt sind seitlich eingeführte Rührwerke. Dabei können Propellerrührwerke über Lanzen seitlich in den Behälter eingeführt werden. Häufig besteht die Möglichkeit, die Lanze mit dem Propeller an der Spitze in einem gewissen Radius zu schwenken. Der Antrieb kann von außen erfolgen oder ebenfalls an der Spitze der Lanze angeordnet sein. Ebenfalls möglich ist es, die Lanze dabei in dem Behälter ein zweites Mal zu lagern und die Rührpropeller auf der Welle anzuordnen. Eine Schwenkfunktion ist dann allerdings nicht mehr vorgesehen. Eine solche Ausgestaltung ist beispielsweise bekannt aus EP 1 619 239 A2. Paddelrührwerke können auch seitlich eingeführt werden, haben aber in der Regel im Inneren des Behälters einen zweiten Lagerbock mit einem Widerlager. Bekannt sind solche Rührwerke beispielsweise aus DE 20 2008 010 638 U1 und WO 2007/110064 A1. Weiterhin bekannt sind in dem Innenraum eines Behälters befestigte Rührwerke. Ein häufig eingesetzter Aufbau besteht darin, Tauchmotorrührwerke mit einem Propeller direkt in dem Behälter zu montieren. Für die Halterung bestehen diverse Möglichkeiten. Häufig verwendet werden Halterungen bestehend aus einem Rührwerksmast, der parallel zu der Außenwand des Behälters gelagert wird und vertikal im Innenraum des Behälters steht. An dem Rührwerksmast wird über einen Lagerbock ein Tauchmotorrührwerk befestigt, welches über einen Seilzug in der Höhe verstellbar ist. Das Rührwerk ist über den um seine Längsachse drehbaren Rührwerksmast schwenkbar. Sowohl die Höhenverstellung als auch das Schwenken können von außen bedient werden.

Um insbesondere an den Rührwerkssystemen eine Wartung vornehmen zu können, muss der Behälter in der Regel geöffnet werden. Gemäß DE 197 32 198 C1, DE 197 14 342 C1 und DE 20 2004 011 561 U1 sind dazu Klappen vorgesehen, durch die eine Wartung erfolgen kann. Auch möglich sind Aussparungen in der Betonhülle des Behälters. Teilweise ist es dabei erforderlich, den Behälter für eine Wartung des Rührwerks teilweise oder vollständig zu entleeren. Dies ist unerwünscht. Außerdem tritt bei den bekannten Systemen im Zuge der Wartung Gas aus dem Behälter aus. Dies ist hinsichtlich Umwelt- und Arbeitsschutz, jedoch auch in Hinsicht auf immer größer werdende Fermentervolumen aus betriebswirtschaftlicher Sicht immer weniger zu tolerieren.

In EP 1 992 405 A1 wird vorgeschlagen, eine Kammer an der Behälterwand anzuordnen, in welche ein Förderelement rückziehbar ist und welche nach dem Rückziehen des Förderelementes flüssigkeits- oder fluiddicht abschließbar ist. Die Kammer wird über ein zwischen der Kammer und dem Behälterinnenraum angeordnetes Absperrelement in Form eines Schiebers geöffnet oder geschlossen. Die Kammer kann durch eine am Behälter befestigte Befestigungsvorrichtung gebildet sein, an welcher wiederum die Fördereinrichtung befestigt ist. Die Befestigungsvorrichtung umfasst eine Zwischenplatte, in der eine Antriebswelle der Fördereinrichtung geführt ist. Auf ihrer dem Behälter abgewandten Seite ist die Zwischenplatte mit einem Motor der Fördereinrichtung durch Schrauben verbunden. Durch Lösen der Schrauben kann die Fördereinrichtung dann von der Befestigungsvorrichtung getrennt werden und ein Propeller der Fördereinrichtung in die durch die Zwischenplatte, ein Anschlusselement und den Schieber begrenzte Kammer zurückgezogen werden. Diese Vorrichtung ist jedoch konstruktiv aufwendig und damit teuer. Außerdem ist die Bedienung dieser Vorrichtung aufwendig. Hinzu kommt, dass die bekannte Vorrichtung gemeinsam mit der Fördereinrichtung lokal fixiert an der Behälterwand angeordnet ist und eine geringe Flexibilität hinsichtlich der Wartung unterschiedlicher Rührwerkstypen besitzt.

Ausgehend von dem erläuterten Stand der Technik liegt der Erfindung die Aufgabe zugrunde, einen Behälter und ein Verfahren der eingangs genannten Art bereitzustellen, mit denen eine Komponente, beispielsweise ein Rührwerk, z.B. für eine Wartung in flexibler Weise und typübergreifend ohne Beeinträchtigung des Anlagenbetriebs, und ohne dass Gas in größeren Mengen austreten kann, aus dem Behälter entnommen werden kann,.

Diese Aufgabe wird erfindungsgemäß durch die Gegenstände der Ansprüche 1 und 7 gelöst. Vorteilhafte Ausgestaltungen finden sich in den abhängigen Ansprüchen, der Beschreibung und den Figuren.

Für einen Behälter der eingangs genannten Art löst die Erfindung die Aufgabe dadurch,
- dass die Entnahmeeinrichtung einen flexiblen Schlauch aufweist, der mit seinem ersten Ende an der Entnahmeöffnung, beispielsweise einer Wartungsöffnung, derart befestigbar ist, dass zwischen dem Behälterinnenraum und dem Schlauchinnenraum eine nach außen fluiddichte Verbindung besteht, durch die die Komponente zumindest teilweise in den Schlauch geführt werden kann,
- dass die Entnahmeeinrichtung eine Verschlussvorrichtung aufweist, mit der der Schlauch im an der Entnahmeöffnung befestigten Zustand fluiddicht verschlossen werden kann, und
- dass die Entnahmeeinrichtung eine Abschnüreinrichtung aufweist, mit der der Schlauch bei zumindest teilweise in den Schlauch eingeführter Komponente im Bereich seines dem Behälter zugewandten ersten Endes unter Verringerung des Schlauchquerschnitts im an der Entnahmeöffnung befestigten Zustand abschnürbar ist, so dass bei geschlossenem Schlauch in dem Schlauchinnenraum ein die Komponente zumindest teilweise aufnehmender, gegenüber der Umgebung fluiddichter Raum entsteht.

Für ein Verfahren der eingangs genannten Art löst die Erfindung die Aufgabe durch die Schritte:
- ein flexibler Schlauch wird mit seinem ersten Ende derart an der Entnahmeöffnung befestigt, dass zwischen dem Behälterinnenraum und dem Schlauchinnenraum eine gegenüber der Umgebung fluiddichte Verbindung besteht,
- der Schlauch wird fluiddicht verschlossen,
- die Komponente wird durch die Entnahmeöffnung zumindest teilweise in den Schlauch geführt,
- der Schlauch wird im Bereich seines dem Behälter zugewandten ersten Endes bei zumindest teilweise in den Schlauch eingeführter Komponente unter Verringerung des Schlauchquerschnitts abgeschnürt, so dass in dem Schlauch ein die Komponente zumindest teilweise aufnehmender, gegenüber der Umgebung fluiddichter Raum entsteht, und
- der Schlauch wird geöffnet, so dass die sich zumindest teilweise in dem Schlauch befindliche Komponente zugänglich ist, beispielsweise gewartet werden kann.

Es ist möglich, dass der Schlauch eine zweite Öffnung aufweist, die zum Verschließen des Schlauchs mit der Verschlussvorrichtung im an der Entnahmeöffnung befestigten Zustand fluiddicht verschlossen werden kann. Weiter kann mit der Abschnüreinrichtung der Schlauch derart abschnürbar sein, dass bei geschlossener zweiter Öffnung in dem Schlauchinnenraum der die Komponente zumindest teilweise aufnehmende, gegenüber der Umgebung fluiddichte Raum entsteht. Als zweite Öffnung ist beispielsweise eine in Längsrichtung des Schlauches verlaufende Öffnung denkbar, die beispielsweise mittels eines Reißverschlusses oder einer anderen Verschlussvorrichtung zu öffnen und zu schließen sein kann.

Weiterhin ist es möglich, dass zum Verschließen des Schlauchs mit der Verschlussvorrichtung das dem Behälterinnenraum abgewandte zweite Ende des Schlauchs im an der Entnahmeöffnung befestigten Zustand fluiddicht verschlossen werden kann. Entsprechend kann dann mit der Abschnüreinrichtung der Schlauch derart abschnürbar sein, dass bei geschlossenem zweiten Ende in dem Schlauchinnenraum der die Komponente zumindest teilweise aufnehmende, gegenüber der Umgebung fluiddichte Raum entsteht.

Sofern es sich bei der Komponente um ein Rührwerk handelt, kann dieses samt seinem Halter aus dem Behälter entnommen werden. Der erfindungsgemäße Behälter kann insbesondere ein Fermentations- oder Lagerbehälter sein. Entsprechend kann das Substrat ein Gärsubstrat sein. Der Behälter besitzt eine Entnahmeöffnung, beispielsweise eine Wartungsöffnung, durch die die Komponente, z.B. das Rührwerk, zu Wartungszwecken oder anderen Zwecken entnommen werden kann. Im Betrieb der Anlage kann die Entnahmeöffnung z.B. mittels einer Klappe oder ähnlichem verschlossen werden. Zur Wartung wird die Klappe geöffnet. Zur Entnahme der Komponente aus dem Behälter zu Wartungszwecken ist erfindungsgemäß eine Entnahmeeinrichtung vorgesehen, die einen Schlauch aus einer flexiblen Folie, z.B. einer Kunststofffolie aufweist. Der Schlauch kann zunächst an beiden Enden offen sein. Mit seinem einen Ende kann der Schlauch dann an der Entnahmeöffnung befestigt sein. Mittels der Verschlussvorrichtung kann der Schlauch, insbesondere eine zweite Öffnung oder das im Montagezustand dem Behälterinnenraum abgewandte Schlauchende, fluiddicht verschlossen und insbesondere auch wieder geöffnet werden. Natürlich könnte dieses Schlauchende auch z.B. bei der Herstellung (permanent) verschlossen werden und der Schlauch zum Öffnen dieses Endes z.B. zerschnitten werden. Bevorzugt ist jedoch eine Verschlussvorrichtung, mit der der Schlauch, insbesondere eine zweite Öffnung oder das zweite Ende des Schlauchs, mehrfach verschlossen und wieder geöffnet werden kann. In Frage kommen z.B. ein gasdichter Reißverschluss oder ein Klammerverschluss. Es wird darauf hingewiesen, dass der Schlauch, insbesondere eine zweite Öffnung oder das dem Behälterinnenraum abgewandte Ende des Schlauchs, vor oder nach der Befestigung des Schlauchs an der Entnahmeöffnung fluiddicht verschlossen werden kann. Fluiddicht verschlossen bedeutet, dass durch den Schlauch, insbesondere eine zweite Öffnung oder das betreffende Schlauchende, kein Fluid fließen kann. Es ist dabei auch möglich, dass noch ein Teil der zu entnehmenden Komponente durch das fluiddicht verschlossene Ende ragt, der Schlauch sich beim Verschließen jedoch an den Umfang dieses Teils, z.B. eine Antriebswelle eines Rührwerks, dicht anlegt.

Nach dem Verschließen des Schlauchs, insbesondere der zweiten Öffnung oder des zweiten Schlauchendes, und dem Befestigen des Schlauchs an der Entnahmeöffnung kann die Komponente zumindest teilweise aus dem Behälterinnenraum in den Schlauch geführt werden und auch das dem Behälter zugewandte erste Ende fluiddicht abgeschnürt werden, so dass auch durch dieses Ende kein Fluid mehr fließen kann. Sofern die Komponente ein Rührwerk ist, kann dieses mit oder ohne seinen Halter, im Ganzen jedoch zumindest teilweise in den Schlauch geführt werden. Zum Abschnüren des ersten Schlauchendes ist erfindungsgemäß eine Abschnüreinrichtung vorgesehen. Sofern sich die zu entnehmende Komponente vollständig in dem Schlauch befindet, wird der Schlauchquerschnitt entsprechend vollständig abgeschnürt. Sofern sich dagegen ein Teil der Komponente noch in dem Behälter befindet, wird der Schlauch um den aus dem Behälter ragenden Teil der Komponente, z.B. eine Antriebswelle oder ein Halter eines Rührwerks, abgeschnürt, wobei sich der Schlauch dicht an dieses Teil anlegt und so die Abdichtung des Schlauchinnenraums gegenüber der Umgebung bewerkstelligt. Mit der Abschnüreinrichtung kann die Abdichtung dabei in besonders einfacher Weise erreicht werden. Durch Lösen der Abschnürung kann das Schlauchende wiederum in besonders einfacher Weise wieder geöffnet werden. Nach dem Abschnüren des ersten Endes wird der Schlauch, insbesondere seine zweite Öffnung oder das zweite Ende des Schlauchs, geöffnet, so dass die in dem Schlauch befindliche Komponente einer Wartung o.ä. zugänglich ist.

Der Schlauch bildet also eine Schleuse zum Ausschleusen der Komponente aus dem Behälterinnenraum, durch die der Übergang von dem Behälterinnenraum in die Umgebung ermöglicht wird, wobei beide Räume stetig voneinander getrennt sind. Dabei kommt es nur zu einem minimalen Entweichen von Biogas aus dem Behälter, da das erste Schlauchende fluiddicht abgeschnürt ist. Insbesondere kann nur das in dem Schlauch befindliche Gasvolumen entweichen. Der Betrieb der Biogasanlage wird erfindungsgemäß also praktisch nicht beeinflusst. Gleichzeitig ist durch den Einsatz einer Abschnüreinrichtung und eines flexiblen Schlauchs das Verschließen und Öffnen des Schlauchs hinsichtlich Konstruktion und Bedienung besonders einfach und damit auch kostengünstig. Aufwendige Befestigungseinrichtungen, wie beim Stand der Technik, sind erfindungsgemäß nicht erforderlich.

Weiterhin ist die zu entnehmende Komponente erfindungsgemäß nicht fest mit der Entnahmeeinrichtung verbunden. Daher und aufgrund der einfachen Montierbarkeit ist die erfindungsgemäße Entnahmeeinrichtung in flexibler Weise für unterschiedlichste Typen von Komponenten, z.B. Rührwerken, und Behältern einsetzbar. Dies gilt unabhängig von der Größe der Komponenten oder davon, ob es sich beispielsweise um Rührwerke mit in dem Behälter oder außerhalb des Behälters angeordneter Antriebseinheit handelt. Weiterhin gilt dies beispielsweise unabhängig davon, ob z.B. ein Rührwerk ein seitlich oder von oben in den Behälter eingeführtes Rührwerk ist, oder ob Teile des Rührwerks in dem Behälter beweglich, z.B. verschiebbar oder verschwenkbar, sind. Selbstverständlich können bei dem erfindungsgemäßen Behälter auch mehrere Komponenten, wie Rührwerke, vorgesehen sein. Entsprechend können auch mehrere Entnahmeeinrichtungen vorgesehen sein.

Gemäß einer weiteren Ausgestaltung kann der Schlauch lösbar an der Entnahmeöffnung befestigbar sein bzw. befestigt werden. Dadurch kann der Schlauch im normalen Betrieb entfernt werden und muss nur für die Wartung an dem Behälter befestigt werden. Es ist dann auch möglich, in flexibler Weise dieselbe Entnahmeeinrichtung für eine Wartung von Komponenten unterschiedlicher Behälter einzusetzen. Gemäß einer weiteren, besonders praxisgemäßen Ausgestaltung kann der Schlauch mittels einer Schweißverbindung an dem Rand der Entnahmeöffnung befestigbar sein.

Die Verschlussvorrichtung kann ebenfalls eine Abschnüreinrichtung sein, mit der der Schlauch, insbesondere im Bereich seines zweiten Endes, unter Verringerung des Schlauchquerschnitts fluiddicht abschnürbar ist. Durch ein Lösen der Abschnürung kann der Schlauch, insbesondere das zweite Ende, wieder geöffnet werden. Auf diese Weise ist die Verschlussvorrichtung konstruktiv besonders einfach ausgebildet und damit einfach zu bedienen und kostengünstig. Diese Ausgestaltung ist besonders vorteilhaft, wenn die Komponente teilweise aus dem Behälter ragt und das zweite Ende des Schlauchs um diesen Teil der Komponente verschlossen wird, also im fluiddicht verschlossenen Zustand die Komponente noch teilweise aus dem zweiten Schlauchende herausragt.

Die erfindungsgemäß eingesetzten Abschnüreinrichtungen können ein Abschnürband oder eine Abschnürklemme aufweisen. Beispielsweise mit einem Abschnürband können das oder die offenen Enden des Schlauches in besonders einfacher Weise lösbar abgeschnürt werden und so gegen Fluiddurchtritt abgedichtet werden.

Je nach Ausbildung der zu entnehmenden Komponente kann die Entnahmeöffnung in der Behälterwand oder in einer Behälterabdeckung, beispielsweise einer Folienabdeckung, angeordnet sein. Bei derartigen Behältern kommen häufig (Doppel- )Membranfolienabdeckungen zum Einsatz. Sofern die Öffnung in der Folienabdeckung angeordnet ist, kann die Abdeckfolie auch selbst den Schlauch bilden.

Es kann weiterhin eine Evakuiereinrichtung vorgesehen sein, mit der der fluiddichte Raum z.B. über eine Pumpe evakuierbar ist. Es kann dann nach dem Abschnüren des Schlauchs im Bereich seines dem Behälter zugewandten ersten Endes der entstandene fluiddichte Raum z.B. mittels einer Pumpe evakuiert werden. Anschließend kann der Schlauch, insbesondere seine zweite Öffnung oder das dem Behälterinnenraum abgewandte zweite Ende des Schlauchs, geöffnet werden. Durch das Evakuieren schmiegt sich der Schlauch eng an das in dem Raum befindliche Bauteil an. Auf diese Weise wird sichergestellt, dass mit der erfindungsgemäßen Schleuse praktisch kein Gas, in jedem Fall nur eine sehr geringe Gasmenge, aus dem Behälter in die Umgebung gelangen kann.

Beispielsweise bei Rührwerken, bei denen ein Teil des Rührwerks bzw. des Halters im Betrieb aus dem Behälter ragt, kann der flexible Schlauch zunächst mit seinen beiden Enden über den aus dem Behälter ragenden Teil des Rührwerks bzw. der Halters geschoben werden, wobei der Schlauch zusammengeschoben werden kann. Anschließend kann der Schlauch im Bereich seines dem Behälter abgewandten zweiten Endes um den aus dem Behälter ragenden Teil abgeschnürt und das zweite Ende auf diese Weise fluiddicht verschlossen werden. Nach oder vor dem Verschließen des zweiten Endes kann das erste Ende des Schlauchs an dem Rand der Entnahmeöffnung befestigt werden und das Rührwerk anschließend durch die Entnahmeöffnung zumindest teilweise aus dem Behälter geführt werden. Dabei wird der Schlauch wieder auseinander geschoben. Sobald das Rührwerk in dem gewünschten Maß aus dem Behälter geführt wurde, kann der Schlauch im Bereich seines ersten Endes abgeschnürt werden, so dass in dem Schlauch ein fluiddichter Raum für das Rührwerk besteht. Danach kann das zweite Ende des Schlauchs geöffnet und das Rührwerk so beispielsweise einer Wartung zugänglich gemacht werden.

Ein Ausführungsbeispiel der Erfindung wird nachfolgend anhand von Figuren näher erläutert. Es zeigen schematisch:
- Figur 1: einen Ausschnitt einer Schnittansicht eines erfindungsgemäßen Behälters gemäß einem ersten Ausführungsbeispiel in drei Betriebszuständen,
- Figur 2: einen Ausschnitt einer Schnittansicht eines erfindungsgemäßen Behälters gemäß einem zweiten Ausführungsbeispiel in vier Betriebszuständen,
- Figur 3: einen Ausschnitt einer Schnittansicht eines erfindungsgemäßen Behälters gemäß einem dritten Ausführungsbeispiel in vier Betriebszuständen.

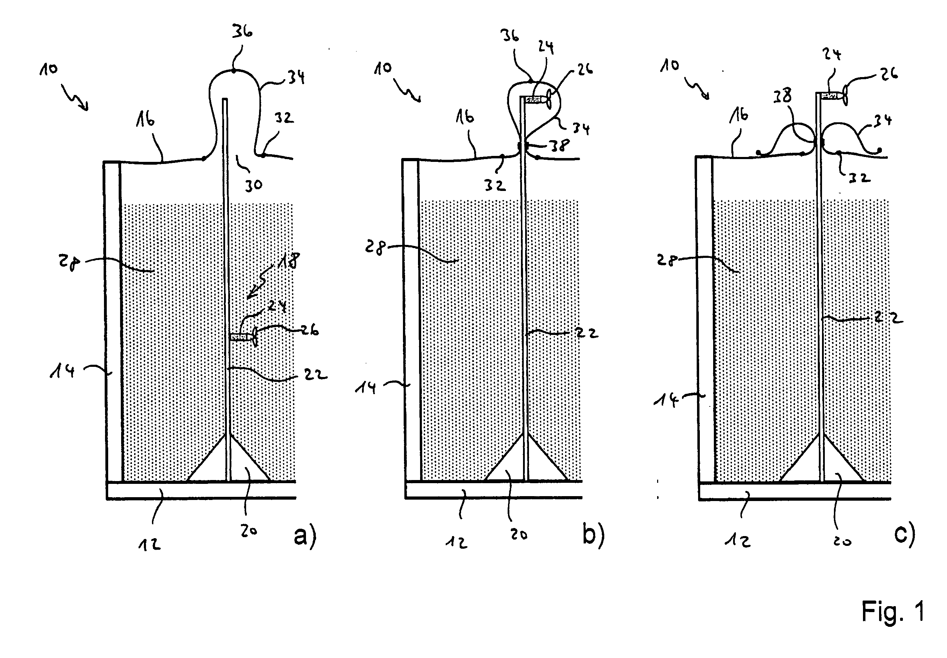

Sofern nichts anderes angegeben ist, bezeichnen in den Figuren gleiche Bezugszeichen gleiche Gegenstände. In Figur 1 ist in den Teilbildern a), b) und c) jeweils ein Ausschnitt eines erfindungsgemäßen Behälters 10 in einer vertikalen Schnittansicht gezeigt. Der Behälter 10 besitzt einen Betonboden 12 und eine im Querschnitt kreisförmig umlaufende Betonwand 14, so dass er insgesamt eine zylindrische Gestalt besitzt. An seinem oberen Ende besitzt der Behälter 10 eine Folienabdeckung 16. Weiterhin besitzt der Behälter 10 in dem in Figur 1 gezeigten Beispiel eine aus dem Behälter entnehmbare Komponente 18, nämlich ein Tauchmotorrührwerk 18 mit einem auf dem Behälterboden 12 durch Stützen 20 abgestützten vertikalen Rührwerksmast 22. An dem Rührwerksmast 22 ist ein Rührwerksantrieb 24 mit einem Propeller 26 höhenverschiebbar gelagert. Über den Antrieb 24 kann der Propeller 26 rotiert werden und so in dem Behälter befindliches Substrat 28 verrührt werden. Die Folienabdeckung 16 ist mit einer Wartungsöffnung 30 versehen. Über eine etwa kreisförmig umlaufende Schweißverbindung 32 ist an dem Rand der Öffnung ein flexibler Schlauch 34 einer Entnahmeeinrichtung zur zumindest teilweisen Entnahme des Rührwerks 18 aus dem Behälter 10 mit seinem ersten Ende befestigt. Sein dem Behälterinnenraum abgewandtes zweites Ende ist durch eine Verschlussvorrichtung 36, in diesem Fall einen gasdichten Reißverschluss oder einen Klammeraufsatz, in den in den Teilbildern a) und b) gezeigten Betriebszuständen fluiddicht verschlossen. Die Schweißverbindung 32 ist ebenfalls fluiddicht, so dass bei dem in a) gezeigten Teilbild in den Schlauchinnenraum Gas aus dem Behälter 10 durch die Öffnung 30 gelangen kann, ohne dass dies in die Umgebung fließen kann.

Zur Wartung des Rührwerks wird bei dem in Figur 1 gezeigten Beispiel das Tauchmotorrührwerk mit seinem Antrieb 24 und seinem Propeller 26 zunächst entlang des Rührwerkmasts 22 vertikal nach oben und durch die Öffnung 30 in den Innenraum des Schlauchs 34 verfahren. Anschließend wird mittels einer Abschnüreinrichtung 38, vorliegend einem Abschnürband 38, der Schlauch 34 im Bereich seines dem Behälterinnenraum zugewandten ersten Endes unter Verringerung des Schlauchquerschnitts um den Rührwerksmast 22 fluiddicht abgeschnürt. Dies ist in Teilbild b) gezeigt. Dadurch ist in dem Schlauch 34 ein den Antrieb 24 und den Propeller 26 des Rührwerks aufnehmender, gegenüber der Umgebung fluiddichter Raum gebildet. Anschließend wird der Schlauch 34 an seinem dem Behälterinnenraum abgewandten zweiten Ende geöffnet, indem die Verschlussvorrichtung 36 gelöst wird. Dadurch werden der Antrieb 24 und der Propeller 26 für eine Wartung freigelegt. Dies kann ohne Entleeren des Behälters 10 und insbesondere ohne merklichen Gasaustritt erfolgen. Dies ist in Figur 1 in Teilbild c) dargestellt.

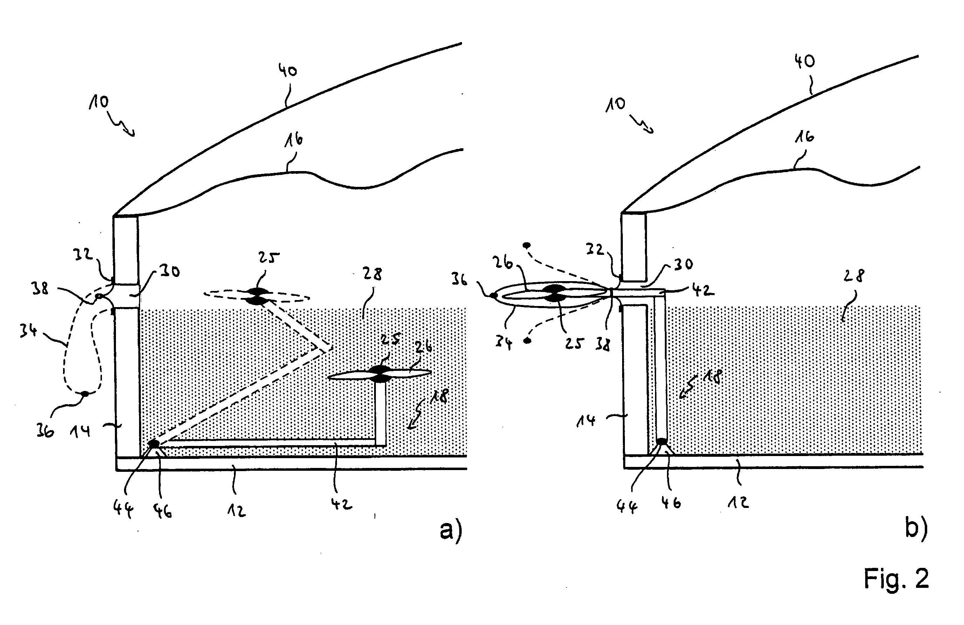

In Figur 2 sind in den Teilbildern a) und b) zwei Betriebszustände eines erfindungsgemäßen Behälters 10 gemäß einem weiteren Ausführungsbeispiel gezeigt. Grundsätzlich entspricht dieser Behälter 10 dem in Figur 1 gezeigten Behälter 10. Im Unterschied zu dem Behälter aus Figur 1 ist in Figur 2 jedoch zusätzlich eine äußere Folienabdeckung 40 des Behälters gezeigt. Weiterhin ist im Unterschied zu Figur 1 bei dem Behälter nach Figur 2 eine Wartungsöffnung 30 in der seitlichen Behälterwand 14 vorgesehen. Das Rührwerk 18 gemäß Figur 2 ist ebenfalls im Unterschied zu der Ausgestaltung nach Figur 1 am Rand des Behälterbodens 12 gelagert und besitzt einen über einen Antrieb 25 antreibbaren Propeller 26, der an einem Ende eines L-förmigen Schwenkarms 42 gehalten wird. An seinem anderen Ende ist der Schwenkarm 42 über ein Schwenklager 44 an einer Bodenabstützung 46 schwenkbar gelagert.

Für eine Wartung des Rührwerks kann an dem äußeren Rand der Wartungsöffnung 30 ein flexibler Folienschlauch 34 mit seinem einen Ende fluiddicht befestigt werden, beispielsweise mittels einer Schweißverbindung 32. Im normalen Betrieb des Behälters 10 kann der Folienschlauch 34 verpackt, z.B. aufgerollt werden. Für eine Wartung wird der Schlauch 34 dann entpackt, wie dies durch die gestrichelte Darstellung des Schlauchs 34 in Teilbild a) veranschaulicht ist. Der Schlauch 34 ist dabei über die Schweißverbindung 32 fluiddicht am Rand der Wartungsöffnung 30 angebracht. Das gegenüberliegende zweite Schlauchende wird wiederum durch eine Verschlussvorrichtung 36 in Form beispielsweise eines Reißverschlusses oder eines Klammeraufsatzes fluiddicht verschlossen. Anschließend wird der Propeller 26 des Rührwerks 18 mittels des Schwenkarms 42 in Richtung zu der Wartungsöffnung 30 verschwenkt, wie dies in Teilbild a) gestrichelt veranschaulicht ist. Durch eine geeignete Drehung des Propellers 26 und ein weiteres Verschwenken des Schwenkarms 42 kann der Propeller 26 anschließend durch die Warlungsöffnung 30 hindurch in den Folienschlauch 34 geführt werden. Anschließend kann mittels eines Abschnürbands 38 das dem Behälterinnenraum zugewandte erste Ende des Schlauchs 34 um den Schwenkmast 42 herum fluiddicht abgeschnürt werden. Dieser Betriebszustand ist in Teilbild b) von Figur 2 gezeigt. In dem Schlauch 34 ist nun ein fluiddicht abgeschlossener Raum gebildet, der den Propeller 26 aufnimmt. Danach kann wiederum die Verschlussrichtung 36 geöffnet werden, so dass der Propeller 26 einer Wartung zugänglich ist.

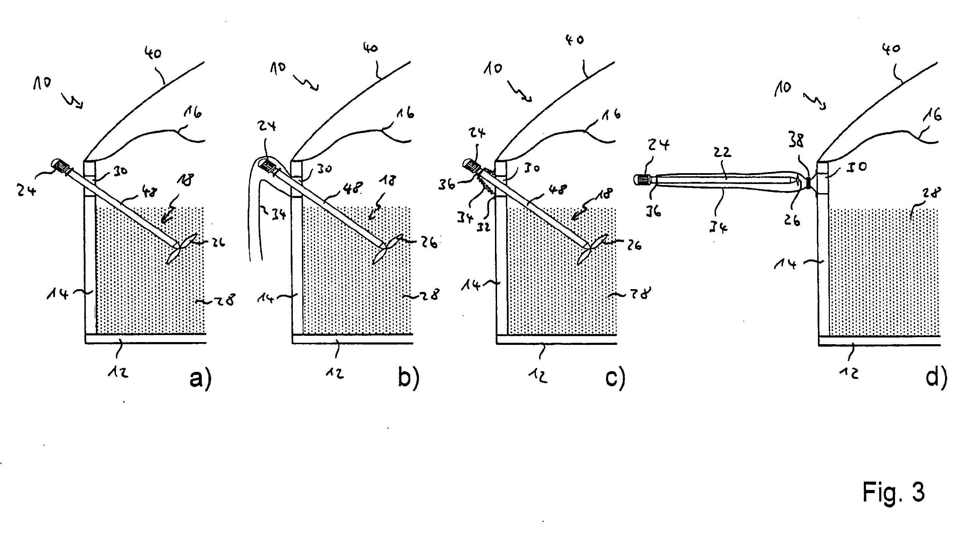

In Figur 3 sind in den Teilbildern a), b), c) und d) vier Betriebszustände eines Behälters 10 gemäß einem dritten Ausführungsbeispiel gezeigt. Der Behälter 10 gemäß Figur 3 entspricht weitgehend dem in Figur 2 dargestellten Behälter 10. Allerdings unterscheidet sich das Rührwerk 18 gemäß Figur 3 von dem in Figur 2 gezeigten Rührwerk. So besitzt das Rührwerk 18 nach Figur 3 eine in dem in Teilbild a) gezeigten Betriebszustand durch die Wartungsöffnung 30 hindurch unter einem Winkel zur Vertikalen in den Behälterinnenraum und insbesondere in das Substrat 28 verlaufende Antriebswelle 48, die an ihrem einen Ende einen Rührpropeller 26 aufweist. In dem in Teilbild a) gezeigten Betriebszustand ragt das Rührwerk 18, und insbesondere die Antriebswelle 48, durch die Wartungsöffnung 30 und damit durch die Behälterwand 14 aus dem Behälter 10 heraus. An ihrer dem Propeller 26 abgewandten Seite trägt die Antriebswelle 48 einen Rührwerksantrieb 24, über den der Propeller 26 zum Mischen des Substrats 28 antreibbar ist. Es handelt sich also um ein seitlich durch die Wand geführtes Lanzenrührwerk mit außen liegendem Antriebsaggregat. Zur Wartung dieses Rührwerks 18 kann ein im normalen Betrieb nicht an dem Behälter 10 montierter Folienschlauch 34 von außen zunächst über den aus dem Behälter 10 herausragenden Teil des Rührwerks 48, also insbesondere die Antriebseinrichtung 24 und einen Teil der Antriebswelle 48, gezogen werden. Dies ist in Figur 3 in Teilbild b) gezeigt. Der Schlauch 34 wird dabei mit seinen beiden offenen Enden vollständig über den aus dem Behälter 10 herausragenden Teil des Rührwerks 18 gezogen. In diesem zusammengeschobenen Zustand des Schlauchs 34 wird er zum einen über eine Schweißverbindung 32 fluiddicht mit dem Rand der Wartungsöffnung 30 verbunden. Zum anderen wird mittels einer als Abschnüreinrichtung ausgebildeten Verschlussvorrichtung 36 das dem Behälter abgewandte Ende des Schlauchs 34 um die Antriebswelle 48 fluiddicht abgeschnürt. Dieser Zustand ist in Teilbild c) gezeigt. Anschließend kann mittels einer nicht dargestellten Hebevorrichtung das Rührwerk 18 durch die Wartungsöffnung 30 aus dem Behälter in die in Figur 3 in Teilbild d) gezeigte Stellung gezogen werden. Dabei wird der Schlauch 34 wieder auseinander geschoben. Sobald das Rührwerk 18 vollständig durch die Wartungsöffnung 30 aus dem Behälter 10 gezogen wurde, wird wiederum mittels einer Abschnüreinrichtung 38 der Schlauch 34 im Bereich seines dem Behälter 10 zugewandten ersten Endes vollständig abgeschnürt. Anschließend kann die Verschlussvorrichtung 36 geöffnet werden und das Rührwerk 18 für die Wartung entnommen werden.

Erfindungsgemäß ist die Wartung eines Rührwerks in besonders einfacher und damit kostengünstiger Weise ohne Beeinträchtigung des Betriebs der Biogasanlage und bei großer Flexibilität hinsichtlich unterschiedlicher Rührwerks- und Behältertypen möglich.

## Patentansprüche

1. Behälter einer Biogasanlage, umfassend eine aus dem Behälter entnehmbare Komponente (18), beispielsweise ein Rührwerk (18) zum Mischen von in dem Behälter (10) befindlichem Substrat (28), eine Entnahmeöffnung (30), sowie eine Entnahmeeinrichtung, mit der die Komponente (18) beispielsweise für Wartungszwecke zumindest teilweise durch die Entnahmeöffnung (30) aus dem Behälter (10) entnommen werden kann, **dadurch gekennzeichnet,**
- **dass** die Entnahmeeinrichtung einen flexiblen Schlauch (34) aufweist, der mit seinem ersten Ende an der Entnahmeöffnung (30) derart befestigbar ist, dass zwischen dem Behälterinnenraum und dem Schlauchinnenraum eine nach außen fluiddichte Verbindung besteht, durch die die Komponente (18) zumindest teilweise in den Schlauch (34) geführt werden kann,
- **dass** die Entnahmeeinrichtung eine Verschlussvorrichtung (36) aufweist, mit der der Schlauch (34) im an der Entnahmeöffnung (30) befestigten Zustand fluiddicht verschlossen werden kann, und
- **dass** die Entnahmeeinrichtung eine Abschnüreinrichtung (38) aufweist, mit der der Schlauch (34) bei zumindest teilweise in den Schlauch (34) eingeführter Komponente (18) im Bereich seines dem Behälter (10) zugewandten ersten Endes unter Verringerung des Schlauchquerschnitts im an der Entnahmeöffnung (30) befestigten Zustand abschnürbar ist, so dass bei geschlossenem Schlauch in dem Schlauchinnenraum ein die Komponente (18) zumindest teilweise aufnehmender, gegenüber der Umgebung fluiddichter Raum entsteht.

2. Behälter nach Anspruch 1, **dadurch gekennzeichnet, dass** der Schlauch (34) eine zweite Öffnung aufweist, die mit der Verschlussvorrichtung (36) im an der Entnahmeöffnung (30) befestigten Zustand fluiddicht verschlossen werden kann und, dass mit der Abschnüreinrichtung (38) der Schlauch (34) bei zumindest teilweise in den Schlauch (34) eingeführter Komponente (18) im Bereich seines dem Behälter (10) zugewandten ersten Endes unter Verringerung des Schlauchquerschnitts im an der Entnahmeöffnung (30) befestigten Zustand abschnürbar ist, so dass bei geschlossener zweiter Öffnung in dem Schlauchinnenraum der die Komponente (18) zumindest teilweise aufnehmende, gegenüber der Umgebung fluiddichte Raum entsteht.

3. Behälter nach einem der vorhergehenden Ansprüche, **dadurch gekennzeichnet, dass** mit der Verschlussvorrichtung (36) das dem Behälterinnenraum abgewandte zweite Ende des Schlauchs im an der Entnahmeöffnung (30) befestigten Zustand fluiddicht verschlossen werden kann und, dass mit der Abschnüreinrichtung (38) der Schlauch (34) bei zumindest teilweise in den Schlauch (34) eingeführter Komponente (18) im Bereich seines dem Behälter (10) zugewandten ersten Endes unter Verringerung des Schlauchquerschnitts im an der Entnahmeöffnung (30) befestigten Zustand abschnürbar ist, so dass bei geschlossenem zweiten Ende in dem Schlauchinnenraum der die Komponente (18) zumindest teilweise aufnehmende, gegenüber der Umgebung fluiddichte Raum entsteht.

4. Behälter nach einem der vorhergehenden Ansprüche, **dadurch gekennzeichnet, dass** der Schlauch (34) lösbar an der Entnahmeöffnung (30) befestigbar ist.

5. Behälter nach einem der Ansprüche 1 bis 3, **dadurch gekennzeichnet, dass** der Schlauch (34) mittels einer Schweißverbindung (32) an dem Rand der Entnahmeöffnung (30) befestigbar ist.

6. Behälter nach einem der vorhergehenden Ansprüche, **dadurch gekennzeichnet, dass** die Verschlussvorrichtung (36) ebenfalls eine Abschnüreinrichtung ist, mit der der Schlauch (34) unter Verringerung des Schlauchquerschnitts fluiddicht abschnürbar ist, insbesondere im Bereich seines zweiten Endes.

7. Behälter nach einem der vorhergehenden Ansprüche, **dadurch gekennzeichnet, dass** die Verschlussvorrichtung (36) eine Klemme ist, mit der der Schlauch (34) fluiddicht wiederverschließbar ist, insbesondere im Bereich seines zweiten Endes.

8. Behälter nach einem der vorhergehenden Ansprüche, **dadurch gekennzeichnet, dass** die Verschlussvorrichtung (36) ein gasdichter Reißverschluss ist, mit dem der Schlauch (34) fluiddicht wiederverschließbar ist, insbesondere im Bereich seines zweiten Endes.

9. Behälter nach einem der vorhergehenden Ansprüche, **dadurch gekennzeichnet, dass** eine Evakuiereinrichtung vorgesehen ist, mit der der fluiddichte Raum evakuierbar ist.

10. Behälter nach einem der vorhergehenden Ansprüche, **dadurch gekennzeichnet, dass** die mindestens eine Abschnüreinrichtung (38) ein Abschnürband (38) oder eine Abschnürklemme aufweist.

11. Behälter nach einem der vorhergehenden Ansprüche, **dadurch gekennzeichnet, dass** die Entnahmeöffnung (30) in der Behälterwand (14) oder in einer Behälterabdeckung (16), insbesondere einer Folienabdeckung (16), angeordnet ist.

12. Verfahren zur zumindest teilweisen Entnahme einer aus dem Behälter entnehmbaren Komponente (18), beispielsweise eines Rührwerks (18) zum Mischen von in einem Behälter (10) einer Biogasanlage befindlichem Substrat (28), aus dem Behälter (10) durch eine Entnahmeöffnung (30) des Behälters (10) beispielsweise für Wartungszwecke, umfassend die Schritte:
- ein flexibler Schlauch (34) wird mit seinem ersten Ende derart an der Entnahmeöffnung (30) befestigt, dass zwischen dem Behälterinnenraum und dem Schlauchinnenraum eine gegenüber der Umgebung fluiddichte Verbindung besteht,
- der Schlauch (34) wird fluiddicht verschlossen,
- die Komponente (18) wird durch die Entnahmeöffnung (30) zumindest teilweise in den Schlauch (34) geführt,
- der Schlauch (34) wird im Bereich seines dem Behälter (10) zugewandten ersten Endes bei zumindest teilweise in den Schlauch (34) eingeführter Komponente (18) unter Verringerung des Schlauchquerschnitts abgeschnürt, so dass in dem Schlauch (34) ein die Komponente (18) zumindest teilweise aufnehmender, gegenüber der Umgebung fluiddichter Raum entsteht, und
- der Schlauch (34) wird geöffnet, so dass die sich zumindest teilweise in dem Schlauch (34) befindende Komponente (18) zugänglich ist.

13. Verfahren nach Anspruch 12, **dadurch gekennzeichnet, dass** zum Verschließen des Schlauches (34) eine zweite Öffnung des Schlauches (34) fluiddicht verschlossen wird und, dass zum Öffnen des Schlauchs (34) die zweite Öffnung des Schlauchs (34) geöffnet wird, so dass die sich zumindest teilweise in dem Schlauch (34) befindende Komponente (18) zugänglich ist.

14. Verfahren nach einem der Ansprüche 12 oder 13, **dadurch gekennzeichnet, dass** zum Verschließen des Schlauches (34) das zweite Ende des Schlauches (34) fluiddicht verschlossen wird und, dass zum Öffnen des Schlauchs (34) das dem Behälterinnenraum abgewandte zweite Ende des Schlauchs (34) geöffnet wird, so dass die sich zumindest teilweise in dem Schlauch (34) befindende Komponente (18) zugänglich ist.

15. Verfahren nach einem der Ansprüche 12 bis 14, **dadurch gekennzeichnet, dass** der Schlauch (34) lösbar an der Entnahmeöffnung (30) befestigt wird.

16. Verfahren nach einem der Ansprüche 12 bis 14, **dadurch gekennzeichnet, dass** der Schlauch (34) an dem Rand der Entnahmeöffnung (30) verschweißt wird.

17. Verfahren nach einem der Ansprüche 12 bis 16, **dadurch gekennzeichnet, dass** zum Verschließen des des Schlauchs (34), insbesondere des zweiten Endes des Schlauchs (34), der Schlauch (34), insbesondere im Bereich seines zweiten Endes, unter Verringerung des Schlauchquerschnitts fluiddicht abgeschnürt wird oder mittels einer Klemme oder eines gasdichten Reißverschlusses wiederverschließbar fluiddicht verschlossen wird.

18. Verfahren nach einem der Ansprüche 12 bis 17, **dadurch gekennzeichnet, dass** nach dem Abschnüren des Schlauchs (34) im Bereich seines dem Behälter (10) zugewandten ersten Endes der entstandene fluiddichte Raum evakuiert wird, bevor der Schlauch, insbesondere das dem Behälterinnenraum abgewandte zweite Ende des Schlauchs (34), geöffnet wird.

19. Verfahren nach einem der Ansprüche 12 bis 18, **dadurch gekennzeichnet, dass** die Komponente (18) im Betrieb zumindest teilweise aus dem Behälter (10) ragt und, dass der flexible Schlauch (34) zunächst mit seinen beiden Enden über den aus dem Behälter (10) ragenden Teil der Komponente (18) geschoben wird und anschließend im Bereich seines zweiten Endes um den aus dem Behälter (10) ragenden Teil abgeschnürt und das zweite Ende auf diese Weise fluiddicht verschlossen wird.

20. Verfahren nach einem der Ansprüche 12 bis 19, **dadurch gekennzeichnet, dass** die Komponente (18) durch eine Entnahmeöffnung (30) in der Behälterwand (14) oder in einer Behälterabdeckung (16), insbesondere einer Folienabdeckung (16), entnommen wird.

## Claims

1. A container of a biogas plant, comprising a component (18) adapted to be taken out of the container, for instance a stirring unit (18) for mixing substrate (28) present in the container (10), a withdrawal opening (30) and a withdrawal device by which the component (18) can be taken out of the container (10) at least partially through the withdrawal opening (30) for instance for purposes of maintenance, **characterized in**
• **that** the withdrawal device has a flexible tube (34), which is adapted to be attached on the withdrawal opening (30) with its first end such that there is a connection between the interior of the container and the interior of the tube, which is fluid-tight with respect to the outside and through which the component (18) can be introduced at least partially into the tube (34),
• **that** the withdrawal device has a closing equipment (36), by which the tube (34) can be fluid-tightly closed in the condition that it is attached to the withdrawal opening (30), and
• **that** the withdrawal device has a constriction device (38), by which the tube (34) can be constricted in the region of its first end facing the container (10) in the condition of being fixed on the withdrawal opening (30) when the component (18) is a least partially introduced into the tube (34), the cross section of the tube being reduced in this, so that upon closed flexible tube, a space which is fluid-tight with respect to the surroundings is generated in the interior of the tube which at least partly incorporates the component (18).

2. The container according to claim 1, **characterized in that** the tube (34) has a second opening which can be fluid-tightly closed by the closing equipment (36) in the condition of being fixed on the withdrawal opening (30), and that the tube (34) can be constricted by the constriction device (38) in the region of its first end facing the container (10) when the component (18) is a least partially introduced into the tube (34), the cross section of the tube being reduced in this, so that upon closed second opening, the space which is fluid-tight with respect to the surroundings is generated in the interior of the tube which at least partly incorporates the component (18).

3. A container according to any one of the precedent claims, **characterized in that** the second end of the tube facing away from the interior of the container can be fluid-tightly closed by the closing equipment (36) in the condition of being fixed to the withdrawal opening (30), and that the tube (34) can be constricted by the constriction device (38) in the region of its first end facing the container (10) in the condition of being fixed to the withdrawal opening (30) when the component (18) is a least partially introduced into the tube (34), the cross section of the tube being reduced in this, so that upon closed second end the space which is fluid-tight with respect to the surroundings is generated in the interior of the tube which at least partly incorporates the component (18).

4. A container according to any one of the precedent claims, **characterized in that** the tube (34) is adapted so as to be detachably fixed to the withdrawal opening (30).

5. A container according to any one of claims 1 to 3, **characterized in that** the tube (34) is adapted so as to be fixed on the edge of the withdrawal opening (30) by means of a welded joint (32).

6. A container according to any one of the precedent claims, **characterized in that** the closing equipment (36) is also a constriction device, by which the tube (34) can be fluid-tightly closed, the cross section of the tube being reduced in this, in particular in the region of its second end.

7. A container according to any one of the precedent claims, **characterized in that** the closing equipment (36) is a clamp, by which the tube (34) can be fluid-tightly closed again, in particular in the region of its second end.

8. A container according to any one of the precedent claims, **characterized in that** the closing equipment (36) is a gas-tight zipper, by which the tube (34) can be fluid-tightly closed again, in particular in the region of its second end.

9. A container according to any one of the precedent claims, **characterized in that** an evacuation device is provided by which the fluid-tight space can be evacuated.

10. A container according to any one of the precedent claims, **characterized in that** the at least one constriction device (38) has a constriction tape (38) or a constriction clamp.

11. A container according to any one of the precedent claims, **characterized in that** the withdrawal opening (30) is arranged in the container wall (14) or in a container cover (16), a foil cover (16) in particular.

12. A method for at least partial withdrawal out of the container (10) of a component (18) adapted to be taken out of the container, for instance of a stirring unit (18) for mixing substrate (28) present in a container (10) of a biogas plant through a withdrawal opening (30) of the container (10), for instance for purposes of maintenance, comprising the steps:
• a flexible tube (34) is fixed on the withdrawal opening (30) with its first end, such that a fluid-tight connection with respect to the surroundings exists between the interior of the container and the interior of the tube,
• the tube (34) is fluid-tightly closed,
• the component (18) is at least partially introduced into the tube (34) through the withdrawal opening (30),
• the tube (34) is constricted in the region of its first end facing the container (10) when the component (18) is a least partially introduced into tube (34), the cross section of the tube being reduced in this, so that a space fluid-tight with respect to the surroundings is generated in the interior of the tube (34) which at least partly incorporates the component (18), and
• the tube (34) is opened, so that the component (18) at least partially present in the tube (34) is accessible.

13. The method according to claim 12, **characterized in that** a second opening of the tube (34) is fluid-tightly closed in order to close the tube (34), and that for opening the tube (34), the second opening of the tube (34) is opened, so that the component (18) at least partially present in the tube (34) is accessible.

14. A method according to one of claims 12 or 13, **characterized in that** the second end of the tube (34) is fluid-tightly closed in order to close the tube (34), and that for opening the tube (34), the second end of the tube (34) facing away from the interior of the container is opened, so that the component (18) at least partially present in the tube (34) is accessible.

15. A method according to any one of claims 12 to 14, **characterized in that** the tube (34) is detachably fixed to the withdrawal opening (30).

16. A method according to any one of claims 12 to 14, **characterized in that** the tube (34) is welded on the edge of the withdrawal opening (30).

17. A method according to any one of claims 12 to 16, **characterized in that** in order to close the tube (34), in particular the second end of the tube (34), the tube (34) is fluid-tightly constricted in particular in the region of its second end, the cross section of the tube being reduced in this, or is reclosably and fluid-tightly closed by means of a clamp or a gas-tight zipper.

18. A method according to any one of claims 12 to 17, **characterized in that** after the constriction of the tube (34) in the region of its first end facing the container (10), the generated fluid-tight space is evacuated, before the flexible tube, in particular the second end of tube (34) facing away from the interior of the container, will be opened.

19. A method according to any one of claims 12 to 18, **characterized in that** the component (18) projects at least partially out of the container (10) in the operation, and that the flexible tube (34) is at first thrust with its two ends over that part of the component (18) which projects out of the container (10), and is subsequently constricted around the part projecting out of the container (10) in the region of its second end, and the second end is fluid-tightly closed in this way.

20. A method according to any one of claims 12 to 19, **characterized in that** the component (18) is withdrawn through a withdrawal opening (30) in the container wall (14) or in a container covering (16), a foil covering (16) in particular.

## Revendications

1. Récipient d'une installation de biogaz, comportant un composant (18) retirable du récipient, par exemple un agitateur (18) pour mélanger du substrat (28) présent dans le récipient (10), une ouverture de prélèvement (30) et un dispositif de prélèvement par lequel le composant (18) peut être prélevé du récipient (10) au moins partiellement à travers l'ouverture de prélèvement (30) par exemple pour des opérations de maintenance, **caractérisé en ce**
• **que** le dispositif de prélèvement a un tuyau flexible (34), qui peut être fixé sur l'ouverture de prélèvement (30) avec sa première extrémité de sorte qu' il y a une connexion entre l'espace intérieur du récipient et l'espace intérieur du tuyau qui est étanche aux fluides par rapport à l'extérieur et à travers de laquelle le composant (18) peut être introduit au moins partiellement dans le tuyau (34),
• **que** le dispositif de prélèvement a un dispositif de fermeture (36), par lequel le tuyau flexible (34) peut être fermé de manière étanche aux fluides dans la condition qu'il est fixé à l'ouverture de prélèvement (30), et
• **que** le dispositif de prélèvement a un dispositif de constriction (38), par lequel le tuyau (34) peut être serré dans la région de sa première extrémité opposée au récipient (10) dans la condition qu'il est fixé sur l'ouverture de prélèvement (30), le composant (18) étant au moins partiellement introduit au tuyau (34), la section transversale du tuyau étant réduite en cela, de sorte que quand le tuyau est fermé, un espace étanche aux fluides par rapport à l'environnement est généré dans 1' espace intérieur du tuyau qui héberge au moins partiellement le composant (18).

2. Récipient selon la revendication 1, **caractérisé en ce que** le tuyau (34) a une deuxième ouverture qui peut être fermée de manière étanche aux fluides par le dispositif de fermeture (36) dans la condition qu'il est fixé sur l'ouverture de prélèvement (30), et que le tuyau (34) peut être serré par le dispositif de constriction (38) dans la région de sa première extrémité opposée au récipient (10), le composant (18) étant au moins partiellement introduit au tuyau (34), la section transversale du tuyau étant réduite par le serrement, de sorte que la deuxième ouverture étant fermée, l'espace étanche aux fluides par rapport à l'environnement est généré dans l'espace intérieur du tuyau qui au moins partiellement héberge le composant (18).

3. Récipient selon l'une quelconque des revendication précédentes, **caractérisé en ce que** la deuxième extrémité du tuyau, opposée à l'espace intérieur du récipient, peut être fermée de manière étanche aux fluides par le dispositif de fermeture (36) dans la condition qu'elle est fixée à l'ouverture de prélèvement (30), et que le tuyau (34) peut être serré par le dispositif de constriction (38) dans la région de sa première extrémité opposée au récipient (10) dans la condition qu'il est fixé à l'ouverture de prélèvement (30), le composant (18) étant au moins partiellement introduit au tuyau (34), la section transversale du tuyau étant réduite par le serrement, de sorte que la deuxième extrémité étant fermée, l'espace étanche aux fluides par rapport à l'environnement est généré dans l'espace intérieur du tuyau qui au moins partiellement héberge le composant (18).

4. Récipient selon l'une quelconque des revendication précédentes, **caractérisé en ce que** le tuyau (34) peut être fixé de façon détachable sur l'ouverture de prélèvement (30).

5. Récipient selon l'une quelconque des revendications 1 à 3, **caractérisé en ce que** le tuyau (34) peut être fixé sur le bord de l'ouverture de prélèvement (30) moyennant un joint soudé (32).

6. Récipient selon l'une quelconque des revendication précédentes, **caractérisé en ce que** le dispositif de fermeture (36) est aussi un dispositif de constriction, par lequel le tuyau (34) peut être serré de manière étanche aux fluides, la section transversale du tuyau étant diminuée par cela, notamment dans la région de sa deuxième extrémité.

7. Récipient selon l'une quelconque des revendication précédentes, **caractérisé en ce que** le dispositif de fermeture (36) est une borne, par laquelle le tuyau (34) peut être refermé de manière étanche aux fluides, notamment dans la région de sa deuxième extrémité.

8. Récipient selon l'une quelconque des revendication précédentes, **caractérisé en ce que** le dispositif de fermeture (36) est une fermeture à glissière étanche aux gaz, par laquelle le tuyau (34) peut être refermé de manière étanche aux fluides, notamment dans la région de sa deuxième extrémité.

9. Récipient selon l'une quelconque des revendication précédentes, **caractérisé en ce que** un dispositif d'évacuation est pourvu par lequel l'espace étanche aux fluides peut être vidé.

10. Récipient selon l'une quelconque des revendication précédentes, **caractérisé en ce que** l'au moins un dispositif de constriction (38) a un cordon de constriction (38) ou une borne de constriction.

11. Récipient selon l'une quelconque des revendication précédentes, **caractérisé en ce que** l'ouverture de prélèvement (30) est arrangée dans une paroi de récipient (14) ou dans une couverture (16) du récipient, notamment dans une couverture en feuille de matière plastique (16).

12. Procédé pour enlèvement au moins partiel d'un composant (18) retirable du récipient, par exemple un agitateur (18) pour mélanger du substrat (28) présent dans un récipient (10) d'une installation de biogaz en dehors du récipient (10) á travers une ouverture de prélèvement (30) du récipient (10), par exemple pour des opérations de maintenance, comportant les étapes:
• un tuyau flexible (34) est fixé sur l'ouverture de prélèvement (30) avec sa première extrémité de manière qu'une connexion étanche aux fluides par rapport à l'environnement existe entre l'espace intérieur du récipient et l'espace intérieur du tuyau,
• le tuyau (34) est fermé de manière étanche aux fluides,
• le composant (18) est au moins partiellement introduit au tuyau (34) à travers l'ouverture de prélèvement (30),
• le tuyau (34) est serré dans la région de sa première extrémité opposée au récipient (10), le composant (18) étant au moins partiellement introduit au tuyau (34), la section transversale du tuyau étant réduite par le serrement, de sorte qu'un espace étanche aux fluides par rapport à l'environnement est généré dans le tuyau, qui héberge au moins partiellement le composant (18),
• le tuyau (34) est ouvert, de sorte que le composant (18) au moins partiellement présent dans le tuyau (34) soit accessible.

13. Procédé selon la revendication 12, **caractérisé en ce que** pour une deuxième ouverture du tuyau (34) est fermée de manière étanche aux fluides afin de fermer le tuyau (34), et que pour ouvrir le tuyau (34), la deuxième ouverture du tuyau (34) est ouverte, de sorte que le composant (18) au moins partiellement présent dans le tuyau (34) soit accessible.

14. Procédé selon une des revendications 12 ou 13, **caractérisé en ce que** pour fermer le tuyau (34), la deuxième extrémité du tuyau (34) est fermée de manière étanche aux fluides, et que pour ouvrir le tuyau (34), la deuxième extrémité du tuyau (34) opposée à l'espace intérieur du récipient est ouverte, de sorte que le composant (18) au moins partiellement présent dans le tuyau (34) soit accessible.

15. Procédé selon l'une quelconque des revendications 12 à 14, **caractérisé en ce que** le tuyau (34) est fixé de manière détachable sur l'ouverture de prélèvement (30).

16. Procédé selon l'une quelconque des revendications 12 à 14, **caractérisé en ce que** le tuyau (34) est soudé sur le bord de l'ouverture de prélèvement (30).

17. Procédé selon l'une quelconque des revendications 12 à 16, **caractérisé en ce que** pour fermer le tuyau, notamment la deuxième extrémité du tuyau, le tuyau (34) est serré de manière étanche aux fluides notamment dans la région de sa deuxième extrémité, la section transversale du tuyau étant diminuée par cela, ou il est fermé de manière étanche aux fluides et refermable au moyen d'une pince ou une fermeture à glissière étanche aux gaz.

18. Procédé selon l'une quelconque des revendications 12 à 17, **caractérisé en ce que** après le serrement du tuyau (34) dans la région de sa première extrémité opposée au récipient (10), l'espace étanche aux fluides généré est évacué avant que le tuyau, notamment la deuxième extrémité du tuyau (34) opposée à l'espace intérieur du récipient, sera ouverte.

19. Procédé selon l'une quelconque des revendications 12 à 18, **caractérisé en ce que** le composant (18) projette au moins partiellement du récipient (10) dans l'opération, et que le tuyau flexible (34) est tout d'abord poussé avec ses deux extrémités par-dessus de la partie du composant (18) qui projette du récipient (10), et est ensuite serré autour de la partie qui projette du récipient (10) dans la région de sa deuxième extrémité, et la deuxième extrémité est fermée de manière étanche aux fluides de cette façon.

20. Procédé selon l'une quelconque des revendications 12 à 19, **caractérisé en ce que** le composant (18) est prélevé à travers une ouverture de prélèvement (30) dans la paroi (14) du récipient ou dans un couvercle (16) du récipient, notamment une feuille de recouvrement (16).
